# EUROPEAN PATENT APPLICATION

(11) **EP 2 345 663 A1**
(43) Date of publication of application: **20.07.2011**
(21) Application number: 09823526.0
(22) Date of filing: 23.10.2009
(51) Int. Cl.: C07K 11/02, A61K 38/00, A61P 11/00, A61P 11/06

(54) **CYCLIC DEPSIPEPTIDE COMPOUND AND USE THEREOF**

(30) Priority: 27.10.2008 JP 2008274995
(71) Applicant: Astellas Pharma Inc., Tokyo 103-8411 (JP)
(72) Inventor: TSUJIMOTO, Susumu, Tokyo 103-8411 (JP); TANIGUCHI, Masatoshi, Tokyo 103-8411 (JP); HIRAYAMA, Yoshitaka, Tokyo 103-8411 (JP); TAKASAKI, Jun, Tokyo 103-8411 (JP); KAWASAKI, Tomihisa, Tokyo 103-8411 (JP); SAKAMOTO, Kazutoshi, Tokyo 103-8411 (JP); NISHIWAKI, Shinya, Tokyo 103-8411 (JP); KITANAGA, Yukihiro, Tokyo 103-8411 (JP)
(74) Representative: Gille Hrabal Struck Neidlein Prop Roos
(86) International application number: PCT/JP2009/068266
(87) International publication number: WO 2010/050415

(57) **Abstract**

A compound useful for the prevention and/or treatment of chronic obstructive pulmonary disease (COPD) or asthma, and a pharmaceutical composition containing the compound as an active ingredient are provided.

[Means for Solution]

The present inventors have made extensive studies on the pharmacological actions of naturally fermented materials, and as a result, they have found that a cyclic depsipeptide compound derived from a soil bacterium belonging to the genus *Chromobacterium* which is collected in Okutama-machi, Tokyo has both an inhibitory action on airway contraction and an inhibitory action on airway inflammation, and thus, is useful as an agent for preventing or treating COPD or asthma, thereby completing the present invention. That is, the present invention relates to a pharmaceutical composition for preventing and/or treating chronic obstructive pulmonary disease (COPD) or asthma, which contains a cyclic depsipeptide compound or a salt thereof as an active ingredient and is intended to be administered by intratracheal administration, nasal drop administration, or inhalation administration.

## Description

### Technical Field

The present invention relates to a cyclic depsipeptide compound useful as an active ingredient of a pharmaceutical composition, in particular, a pharmaceutical composition for treating chronic obstructive pulmonary disease (COPD) or asthma, and its use for treating COPD or asthma.

### Background Art

COPD and asthma are both diseases involving limited airflow in the airway.

COPD is a disease in which coughing accompanying exertional dyspnea and sputum is a main symptom. When the disease condition worsens, coughing, wheezing, and dyspnea increase, accompanied by increased sputum which has a changed purulent nature. The airway symptoms such as coughing and wheezing are caused by airway contraction and airway inflammation, and airway hypersecretion accompanied therewith. The disease is characterized in that airflow limitation is not completely reversible. The airflow limitation is usually progressive and occurs with an abnormal inflammatory response of the lung to noxious particles or gases (the Global Initiative for Chronic Obstructive Lung Disease in 2006, which will be hereinafter referred to as GOLD). Most sufferers are smokers. It is estimated that COPD will be the third largest cause of mortality in the world in 2020 (Lancet, 349, pp. 1498-1504, 1997). In a survey conducted in 12 countries of adults over the age of 40, it was estimated that COPD prevalence was 10.1% (Lancet, 370, pp. 741-750, 2007). It is estimated that the COPD prevalence in people of the age of 40 or more in Japan is 10.9% (Respiology, 9, pp. 458-465, 2004).

Asthma is a disease characterized by airway hyperresponsiveness and chronic inflammation, and is also a chronic inflammatory disease of the airway showing reversible airway obstruction improved with therapy and having repeatedly occurring airflow limitation due to airway mucosal edema. It shows symptoms such as paroxysmal dyspnea, coughing, and wheezing. Asthma is one type of chronic respiratory disease and occurs in all age groups ranging from children to the elderly. In addition the number of sufferers, regardless of whether in developed countries or developing countries, is tending to increase and the prevalence thereof in Japan is reported to be 4.2% for infants, 4.0% for children, and 1.7% for adults (Health and Welfare Long-Term Chronic Disease Comprehensive Research Project in Heisei 8). The number of people who have died of asthma in Japan is tending to decrease and was 2778 in 2006, which is still high.

In diagnosing COPD and asthma, a volume in one second indicating a degree of airway obstruction (FEV₁: Forced Expiratory Volume in 1 Second) is presented as an indicator. The "volume in one second" is defined as "a volume of exhaled breath during the first one second when a deep breath is exhaled as quickly as possible after inhalation". Typical disease stage classification of COPD is stipulated in GOLD and the stages are classified into the following four stages according to FEV₁: Stage I (mild): FEV₁≥80% predicted value, II (moderate): 50%≤FEV₁<80% predicted value, III (severe): 30%≤FEV₁<50% predicted value, IV (very severe): FEV₁<30% predicted value plus chronic respiratory failure] (GOLD, 2006). The severity of asthma is classified into the following four stages according to the characteristic symptoms and the respiratory functions (FEV₁ and PEF (Peak Expiratory Flow)): Step 1 (mild intermittent type): FEV₁, PEF≥80% (less than 20% variation), Step 2 (mild persistent type) FEV₁, PEF≥80% (20% to 30% variation), Step 3 (moderate persistent type) 60%≤FEV₁, PEF<80% (over 30% variation), and Step 4 (severe persistent type) FEV₁, PEF<60% (over 30% variation) (Asthma Prevention/Management Guidelines, 2006).

For COPD, a bronchodilator is positioned as a representative therapeutic agent for improving the symptoms, and there are known β2 agonists, anticholinergic agents, and theophylline agents.
For asthma, since the basic disease condition is inflammation, an inhaled corticosteroid has been used as a standard treatment agent. However, for airway stenosis, a bronchodilator, in particular, a long-acting inhaled β2 agonist has been used. In addition, a leukotriene receptor antagonist, a theophylline agent, or the like has also been used.

However, it has been confirmed that existing bronchodilators, for example, anticholinergic agents causes thirst and for β2 agonists, it has been confirmed that an additional effect of inhaled corticosteroids is useful. However, it is reported that asthma-related deaths have significantly increased. Further, in treatment with the β2 agonist alone, for example, the progression of airway remodeling is not inhibited and airway reactivity increases, showing a demand for further improvement in terms of effect and safety.
In addition, it is considered that bronchodilators that also have an anti-inflammatory action are useful for treatment of COPD or asthma, but it has not been reported that the existing bronchodilators clearly exhibit an anti-inflammatory action effect in addition to a bronchodilation action and exhibit improvement in pulmonary emphysema and obstructive change.

It is reported that the compound of the formula (IV) is a cyclic depsipeptide derived from a soil bacterium QS3666 strain belonging to the genus *Chromobacterium* which is collected in Okutama-machi, Tokyo and inhibits human platelet aggregation caused by ADP (Patent Citation 1, Non-Patent Citation 1). The absolute steric structure of the compound of the formula (IV) is determined as in the following formula by a Marfey's method and a chiral HPLC analysis method (Non-Patent Citation 2).

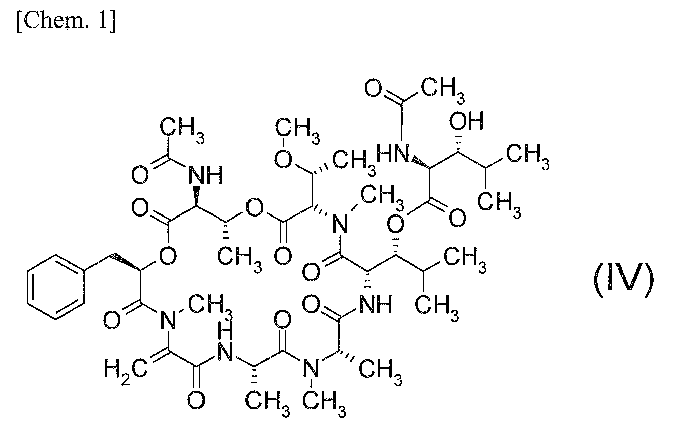

It is reported that the compound of the formula (IV) is a specific G_{q/11} inhibitor (Non-Patent Citation 3). G_{q/11} is one kind of G-protein. The G-protein is a trimer compound consisting of α, β, and γ subunits, and is classified into Gₛ, Gᵢ, G_{q}, and G₁₂ subfamilies in terms of difference in the homology of amino acid sequence and the effector targeted. Further, it is reported that there exist subtypes in the subfamilies, and for G_{q}, there exist subtypes, G_{q}, G₁₁, G₁₄, G₁₅, and G₁₆. The compound of the formula (IV) strongly inhibits G_{q} and G₁₁, but does not inhibit G₁₅ and G₁₆ (Non-Patent Citation 3).

It is reported that the compound of the formula (IV) inhibits the reactions of various receptors, specifically ADP receptors (P2Y1, P2Y2), cysteinyl leukotriene receptors (CryLT-R1, CryLT-R2), and muscarinic receptors (M1) in in-vitro tests (Non-Patent Citation 3).

It is reported that the compound of the formula (IV) exhibits a hypotensive action by intravenously administering it at 30 µg/kg (Non-Patent Citation 4).

In addition, there are reports on the steric structures and the ADP receptor inhibitory actions of the compound of the formula (IV) represented by the following formula (IIa) and analogues thereof (Non-Patent Citation 5).

**[Table 1]**

| | | | | | | | | |
|---|---|---|---|---|---|---|---|---|
| 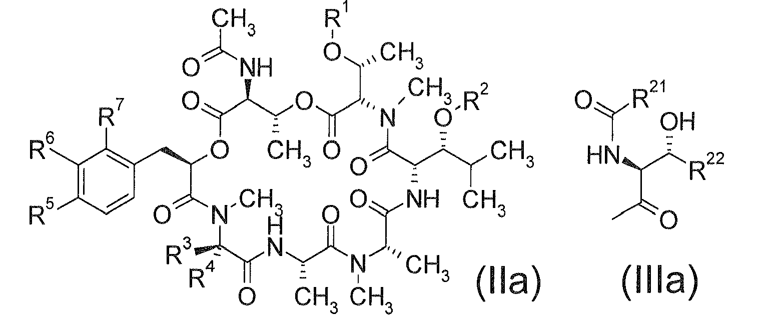 | | | | | | | | |

| R¹ | R² | R²¹ | R²² | R³ | R⁴ | R⁵ | R⁶ | R⁷ |
|---|---|---|---|---|---|---|---|---|
| -CH₃ | (IIIa) | -CH₃ | -CH(CH₃)₂ | =CH₂ | | -H | -H | -H |
| -CH₃ | (IIIa) | -CH₂CH₃ | -CH(CH₃)₂ | =CH₂ | | -H | -H | -H |
| -CH₃ | (IIIa) | -CH₂SCH₃ | -CH(CH₃)₂ | =CH₂ | | -H | -H | -H |
| -CH₃ | -H | - | - | =CH₂ | | -H | -H | -H |
| -CH₃ | (IIIa) | -CH₃ | -CH(CH₃)₂ | =CH₂ | | -H | -H | -F |
| -CH₃ | (IIIa) | -CH₃ | -CH(CH₃)₂ | =CH₂ | | -II | -F | -H |
| -CH₃ | (IIIa) | -CH₃ | -CH(CH₃)₂ | -CH₃ | -H | -H | -H | -H |

It is reported that a linear heptapeptide referred to as a GPAnt-2, which is an N-terminal truncated analogue of Substance P, and an analogue thereof inhibits the hydrolysis of M1 acetylcholine-induced GTP by G_{q/11} by 25% at 10 µM and 81% at 10 µM, respectively (Non-Patent Citation 6). However, since then, there has been no report on a G_{q/11} inhibitor which is selective, in addition to the above cyclic depsipeptides, and a selective inhibitor of G_{q/11} has not been reported, and further confirmation of a further clinical effect of the G_{q/11} inhibitor is expected.

### Related Art Document

### Patent Document

[Patent Citation 1] JP-A-2003-210190

### Non-Patent Document

[Non-Patent Citation 1] Journal of Antibiotics, 56, pp. 358-363, 2003
[Non-Patent Citation 2] Tetrahedron, 59, pp. 4533-4538, 2003
[Non-Patent Citation 3] Journal of Biological Chemistry, 279, pp. 47438-47445, 2004
[Non-Patent Citation 4] Thromb Haemost, 90, pp. 406-413, 2003
[Non-Patent Citation 5] Bioorganic and Medicinal Chemistry, 12, pp. 3125-3133, 2004
[Non-Patent Citation 6] Journal of Biological Chemistry, 267, pp. 16237-16243, 1992

### Disclosure of Invention

### Problems to Be Solved by the Invention

A pharmaceutical useful for treatment of airway obstructive diseases, particularly COPD or asthma, which has both an inhibitory action on airway contraction and an inhibitory action on airway inflammation.

### Means for Solving the Problem

The present inventors have made extensive studies on the pharmacological actions of naturally fermented materials, and as a result, they have found that a cyclic depsipeptide compound represented by the following formula (II) derived from a soil bacterium belonging to the genus *Chromobacterium* which is collected in Okutama-machi, Tokyo has both an inhibitory action on airway contraction and an inhibitory action on airway inflammation, and is effective as an agent for preventing or treating COPD or asthma, thereby completing the present invention.
That is, the present invention relates to a pharmaceutical composition for preventing and/or treating chronic obstructive pulmonary disease (COPD) or asthma, which comprises a cyclic depsipeptide compound of the formula (II) or a salt thereof as an active ingredient and is intended to be administered by intratracheal administration, nasal drop administration, or inhalation administration: 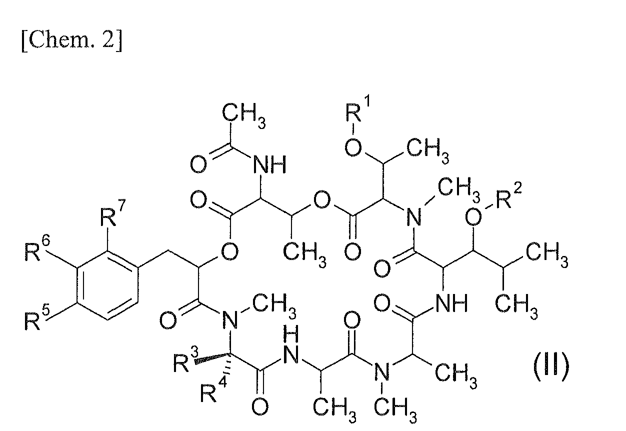 [wherein
R¹ is -H or -CH₃, and
R² is -H or a group represented by the formula (III): 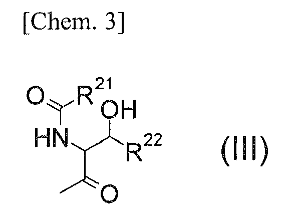 R²¹ is ₋CH₃, -CH₂CH₃, -CH₂CH₂CH₃, or -CH₂SCH₃,
R²² is -CH(CH₃)₂ or -CH₂SCH₃,
R³ and R⁴ are the same as or different from each other, and represent -H or -CH₃, or R³ and R⁴ are combined to form =CH₂, and
R⁵, R⁶, and R⁷ are the same as or different from each other, and represent -H or -F].

Further, the present invention related to a cyclic depsipeptide compound of the formula (II), which administering to a patient by intratracheal administration, nasal drop administration, or inhalation administration, and to a method for preventing or treating a COPD and asthma, which comprises an effective amount of the cyclic depsipeptide compound of the formula (II) or a salt thereof, and which comprises administering to a patient by intratracheal administration, nasal drop administration, or inhalation administration.

Further, the cyclic depsipeptide compound of the formula (II) includes the novel compound of the following formula (I). Accordingly, the present invention also relates to the novel cyclic depsipeptide compound of the formula (I) or a salt thereof: 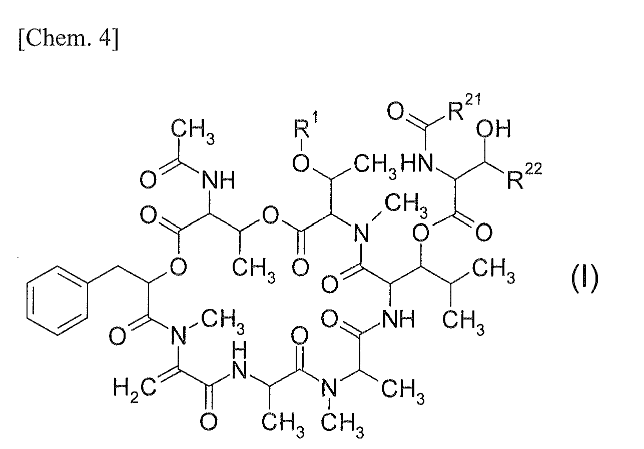 (wherein
R¹ is -CH₃, R²¹ is -CH₂CH₂CH₃, and R²² is -CH(CH₃)₂;
R¹ is -H, R²¹ is -CH₃, and R²² is -CH(CH₃)₂; or
R¹ is -CH₃, R²¹ is -CH₃, and R²² is -CH₂SCH₃).

### Effects of the Invention

The cyclic depsipeptide compound of the formula (II) or a salt thereof has a G_{q/11} inhibitory action, and as shown in Examples as described later, has both an inhibitory action on airway contraction and an inhibitory action on airway inflammation. Accordingly, a pharmaceutical composition containing the compound as an active ingredient is useful as an agent for preventing and/or treating COPD, asthma, or the like.

### Brief Description of Drawings

[FIG. 1] Fig. 1 is a figure showing the percentage change rate in the airway pressure of Ref 1 in Test Example 2. The data are denoted as a mean value+standard error (n=3 or 4). The number in ( ) represents an inhibitory rate for a group administered with saline.
[FIG. 2] Fig. 2 is a figure showing the percentage change rate in airway pressure of Ref 1 in Test Example 3. The data are denoted as a mean value+standard error (n=3). The number in ( ) represents an inhibitory rate for a group administered with saline.
[FIG. 3] Fig. 3 is a figure showing the results of measuring the cell count of Ref 1 in Test Example 5. The data are denoted as a mean value+standard error (n=9 or 10). N, V, 1, 10, and 100 represent a Normal group, a Vehicle group, a 1 µg/kg administration group, a 10 µg/kg administration group, and a 100 µg/kg administration group, respectively. ** represent a significance at p<0.01, as compared with the Vehicle group (multiple comparison of Dunnett).
[FIG. 4] Fig. 4 is a figure showing the ¹H-NMR spectrum of Ex 1 in Preparation Examples.
[FIG. 5] Fig. 5 is a figure showing the ¹³C-NMR spectrum of Ex 1 in Preparation Examples.
[FIG. 6] Fig. 6 is a figure showing the ¹H-NMR spectrum of Ex 2 in Preparation Examples.
[FIG. 7] Fig. 7 is a figure showing the ¹³C-NMR spectrum of Ex 2 in Preparation Examples.
[FIG. 8] Fig. 8 is a figure showing the ¹H-NMR spectrum of Ex 3 in Preparation Examples.
[FIG. 9] Fig. 9 is a figure showing the ¹³C-NMR spectrum of Ex 3 in Preparation Examples.
[FIG. 10] Fig. 10 is a figure showing the ¹H-NMR spectrum of Ex 4 in Preparation Examples.
[FIG. 11] Fig. 11 is a figure showing the ¹³C-NMR spectrum of Ex 4 in Preparation Examples.

### Best Mode for Carrying Out the Invention

Hereinafter, the present invention will be described in detail.

The cyclic depsipeptide compound of the formula (II) of the present invention has a plurality of asymmetric carbon atoms, and correspondingly, it may exist in the form of optical isomers. The cyclic depsipeptide compound which is an active ingredient of the pharmaceutical composition of the present invention includes both an isolated form of the optical isomers of the compound of the formula (II) and a mixture thereof.
Embodiments wherein a cyclic depsipeptide compound represented by the formula (II) or a salt thereof as an active ingredient of the pharmaceutical composition of the present invention are shown below.
(1) The compound or a salt thereof, wherein R² is a group represented by the formula (III).
(2) The compound or a salt thereof, wherein R²¹ is -CH₃ or -CH₂CH₂CH₃.
(3) The compound or a salt thereof, wherein R³ and R⁴ are combined to form =CH₂.
(4) The compound or a salt thereof, wherein R⁵, R⁶, and R⁷ are -H.
(5) A cyclic depsipeptide compound represented by either the formula (IIa) or the formula (I), or a salt thereof.
(6) A cyclic depsipeptide compound represented by the formula (IIa) or a salt thereof.
(7) The compound or a salt thereof, wherein R² is a group represented by the formula (IIIa).
(6) A cyclic depsipeptide compound represented by the formula (I) or a salt thereof.
(7) A cyclic depsipeptide compound represented by the formula (I) or a salt thereof, which has a steric structure characterized by the physical properties shown in Table 3.

As the novel cyclic depsipeptide compound represented by the formula (I) or a salt thereof of the present invention, the depsipeptide compound having a steric structure characterized by the physical properties shown in Table 3 or a salt thereof is preferred.

**[Table 2]**

| | | | | | | | | | |
|---|---|---|---|---|---|---|---|---|---|
| 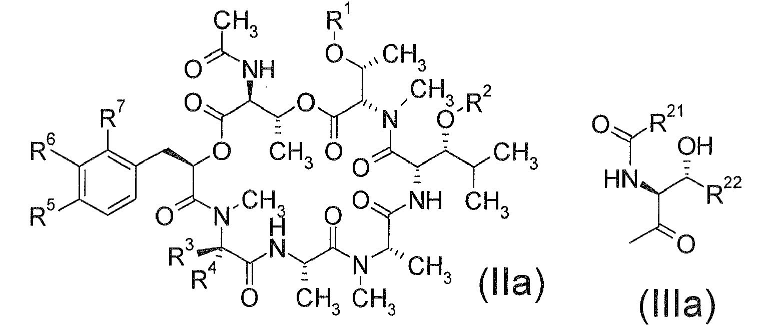 | | | | | | | | | |

| Compound No. | R¹ | R² | R²¹ | R²² | R³ | R⁴ | R⁵ | R⁶ | R⁷ |
|---|---|---|---|---|---|---|---|---|---|
| Ref1 | -CH₃ | (IIIa) | -CH₃ | -CH(CH₃)₂ | =CH₂ | | -H | -H | -H |
| Ref2 | -CH₃ | (IIIa) | -CH₂CH₃ | -CH(CH₃)₂ | =CH₂ | | -H | -H | -H |
| Ref3 | -CH₃ | (IIIa) | -CH₂SCH₃ | -CH(CH₃)₂ | =CH₂ | | -H | -H | -H |
| Ref4 | -CH₃ | -H | - | - | =CH₂ | | -H | -H | -H |
| Ref5 | -CH₃ | (IIIa) | -CH₃ | -CH(CH₃)₂ | =CH₂ | | -H | -H | -F |
| Ref6 | -CH₃ | (IIIa) | -CH₃ | -CH(CH₃)₂ | =CH₂ | | -H | -F | -H |
| Ref7 | -CH₃ | (IIIa) | -CH₃ | -CH(CH₃)₂ | -CH₃ | -H | -H | -H | -H |
| 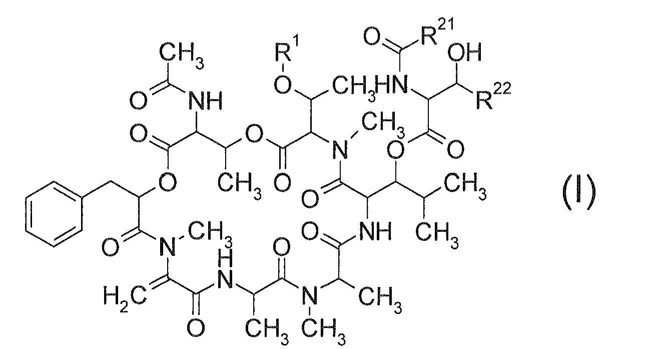 | | | | | | | | | |

| | R¹ | | R²¹ | R²² | | | | | |
|---|---|---|---|---|---|---|---|---|---|
| | -CH₃ | | -CH₂CH₂CH₃ | -CH(CH₃)₂ | | | | | |
| | -H | | -CH₃ | -CH(CH₃)₂ | | | | | |
| | -CH₃ | | -CH₃ | -CH₂SCH₃ | | | | | |

Furthermore, the cyclic depsipeptide compound represented by the formula (IIa) is a compound reported in Non-Patent Citation 5 and Patent Citation 1, and Ref 1 is the compound of the formula (IV) above. Such existing compounds are preferable as an active ingredient of the pharmaceutical composition of the present invention.

In addition, the cyclic depsipeptide compound of the formula (II) or the formula (I) of the present invention includes a pharmaceutically acceptable prodrug thereof. The pharmaceutically acceptable prodrug refers to a compound having a group which can be converted into an amino group, a hydroxyl group, a carboxyl group, or the like by solvolysis or under a physiological condition. Examples of the group for forming a prodrug include those as described in Prog. Med., 5, 2157-2161 (1985) or "Iyakuhin no Kaihatsu (Development of Medicines)" (Hirokawa Shoten, 1990), Vol. 7, Bunshi Sekkei (Molecular Design), 163-198.

Moreover, the salt of the cyclic depsipeptide compound of the formula (II) or the formula (I) is a pharmaceutically acceptable salt, and may form an acid addition salt or a salt with a base, depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like.

In addition, the cyclic depsipeptide compound of the formula (II) or the formula (I) of the present invention or a salt thereof includes various hydrates or solvates, and polymorphic crystal substances of the compound of the present invention and a salt thereof. In addition, the present invention also includes compounds labeled with various radioactive or non-radioactive isotopes.

### (Preparation Methods)

The cyclic depsipeptide compound of the formula (II) or the formula (I) of the present invention is obtained by culturing a strain producing a substance belonging to the genus *Chromobacterium* in a nutrient culture medium, and collecting the cyclic peptide compound from a culture obtained by accumulation of the substance. A desired salt can be obtained by adding a suitable salt-forming reaction.
The microorganism used for preparation of the substance belongs to the genus *Chromobacterium,* and any microorganism having the ability to produce the substance can be used. Examples of the microorganism include a soil bacterium of the genus *Chromobacterium,* a genus *Chromobacterium* sp. QS3666 which is collected in Okutama-machi, Tokyo. This strain is known and its mycological characteristics are described in Patent Citation 1 and deposited with Patent Organism Depositary Center, National Institute of Advanced Industrial Science and Technology (Tsukuba Central 6, 1-1, Higashi 1-chome, Tsukuba-shi, Ibaraki-Ken, Japan (postal code 305-8566)) which is an independent administrative institution (deposit date: January 4, 2002, deposit No. FERM BP-10786). Further, since microorganisms are susceptible to natural or artificial mutation, *for Chromobacterium* sp. QS3666 strain, not only microorganisms isolated from nature may be used, but also ones obtained by artificial mutation such as ultraviolet radiation, radioactive radiation, chemical agents, or the like, and natural variants thereof may also be suitably used as long as the compound of the present invention is allowed to synthesize polymeric materials biologically.

Cultivation is performed according to a conventional cultivation method for microorganisms. As a cuture medium used for culture, any culture media containing nutrients for *Chromobacterium* sp. QS3666 strains can be employed, and synthetic culture media, semi-synthetic culture media, or natural culture media are used. For the composition of the culture media, examples of the carbon source include D-glucose, mannose, maltose, starch, glucose, dextrin, glycerol, vegetable oil, and the like, and examples of the nitrogen source include meat extract, peptone, gluten meal, cotton seed meal, soybean meal, peanut meal, fish meal, corn steep liquor, dry yeast, yeast extract, ammonidum chloride, ammonium sulfate, ammonium nitrate, uric acid, and other organic or inorganic nitrogen sources. Also, if necessary, a metal salt such as the sulfate, nitrate, chloride, carbonate, phosphate, and the like of sodium, potassium, magnesium, calcium, zinc, iron, cobalt, or the like is added to the culture media. Still further, if necessary, a material for promoting the production and a defoaming agent, such as methionine, cysteine, thiosulfate, cystine, methyl oleate, lard oil, silicone oil, a surfactant, and the like may be added.
Among the compounds of the present invention, the compound in which at least one of R⁵, R⁶, and R⁷ is fluorine can be produced according to the conventional cultivation method above, by adding phenylalanine having a fluorine group on a phenyl group into a culture medium.
In regard to the cultivation conditions, it is generally advantageous to cultivate under aerobic conditions, and the cultivation temperature is preferably in a range around from 15 to 32°C, and preferably from 20 to 28°C. Good results are obtained when the pH of the culture medium is adjusted at about 5 to 9, and preferably about 5 to 6. The cultivation period is appropriately set depnding on the composition of the culture medium and the temperature conditions, but is usually around 1 to 20 days, and preferably around 2 to 5 days.

For isolating and purifying the compound of the present invention from the culture, a method for isolating and purifying the biologically active substance from an ordinary culture of microorganisms is applied. That is, a method in which the culture as it is used or mycelium is removed from the culture by centrifugal separation or filtration, and then, such as those utilizing differences in dissolution or solubility in suitable solvents, differences in precipitation properties or precipitation speeds from solutions, differences in adsorptive affinities to various adsorbents, or differences in partition between two liquid phases can be applied. Specific examples thereof include a method in which a culture is brought into contact with a suitable carrier to adsorb the compound in the filtrate, and then purifying the compound by extraction with a suitable solvent. These methods may be, if necessary, used individually or in a combination thereof in any order or further may be repeatedly applied.

The cyclic depsipeptide compound of the formula (II) or the formula (I) of the present invention can also be prepared by any combination of the steps that can usually be employed by a person skilled in the art, such as a known alkylation, acylation, substitution reaction, oxidation, reduction, hydrolysis, deprotection, halogenation, and the like using the corresponding cyclic depsipeptide compound of the formula (II) or the formula (I) of the present invention as a raw material. For example, the compound wherein one of R³ and R⁴ is -CH₃ and the other is -H can be obtained by subjecting the compound wherein R³ and R⁴ are combined to form =CH₂ to catalytic reduction.
For example, the existing compound represented by Ref 7 above can be prepared by subjecting the compound of Ref 1 to catalytic reduction using Pd/C in MeOH as described in p. 3132 of Non-Patent Citation 5.

Specifically, the existing compounds represented by Ref 1 to 7 above were prepared according to the method described in, for example, Patent Citation 1 or Non-Patent Citation 5. Further, novel compounds shown in Ex1 to 4 can be prepared according to the methods described in the Examples as described below. For other compounds included in the present invention, the compounds can be prepared by adjusting the suitable cultivation conditions or separation and purification methods, or by performing a further chemical modification reaction.

A pharmaceutical composition containing one or two or more kinds of the compound of the formula (II) or a salt thereof as an active ingredient is a preparation for intratracheal administration, nasal drop administration, or inhalation administration, using excipients, that is, pharmaceutical excipients, pharmaceutical carriers, or the like, which are usually used in the art, according to the methods usually used.

Regarding making a preparation as a transmucosal agent such as an inhalation and transnasal agent, and the like, the pharmaceutical composition of the present invention is in a solid, liquid, or semi-solid state, and can be prepared in accordance with a conventionally known method. If necessary, a pH adjusting agent, an antiseptic, a surfactant, a lubricant, a stabilizer, a thickener, or the like may be appropriately added thereto.
For their administration, an appropriate device for inhalation or insufflation may be used. For example, a compound may be administered alone or as a powder of a formulated mixture, or as a solution or suspension by combining it with a pharmaceutically acceptable carrier, using a conventionally known device or sprayer, such as DPI (Dry Powder Inhaler), MDI (Metered Dose Inhaler), and the like. The dry powder inhaler or the like may be for single or multiple administration use, and a dry powder or a powder-containing capsule may be used. Alternatively, this may be in the form of a pressurized aerosol spray which uses an appropriate propellant, for example, a suitable gas such as a chlorofluoroalkane, a hydrofluoroalkane, carbon dioxide, and the like.

Usually, in the case of intratracheal, inhalation, or nasal drop administration, the daily dose is suitably from about 0.00001 to 10 mg/kg, and preferably from about 0.001 to 1 mg/kg per body weight, and this is administered once a day or two or more times a day. The dose is appropriately decided in response to an individual case by taking symptoms, age, gender, and the like into consideration.

The compound of the formula (II) can be used in combination with various therapeutic or prophylactic agents, which are usually used for treatment of COPD or asthma. The combined preparation may be administered simultaneously or separately and continuously, or at a desired time interval. The preparations to be co-administered may be a blend, or prepared individually.

### [Examples]

In the present specification, the following abbreviations may be used in some cases. EtOAc = Ethyl acetate, MeOH = Methanol, LTD₄ = Leukotriene D₄, PBS(-) = Phosphate Buffered Saline Containing no Calcium or Magnesium, Methacholine = Acetyl-β-methacholine- chloride.

### Preparation Examples: Preparation Examples for the cyclic depsipeptide compounds Ex 1 to 4 of the formula (I)

### (Cultivation)

The seed medium was prepared by dispensing a culture medium including 5 g of yeast extract, 10 g of polypeptone, 5 g of a salt, and 1 L of distilled water in amounts of 30 mL to each of 100 mL Erlenmeyer flasks, and sterilizing in an autoclave at 121°C for 30 minutes.

The production medium was prepared by dispensing a culture medium (pH 7.0) including 50 g of soybean, 3.3 g of polypeptone, 7.8 g of yeast extract, 20 g of defatted soybean flour, 50 g of a corn steep liquor, 0.65 g of lithium chloride, and 1 L of distilled water in amounts of 30 mL to each of 100-mL Erlenmeyer flasks, and sterilizing in an autoclave at 121°C for 30 minutes.

One platinum loop of a slant culture of *Chromobacterium* sp. QS3666 strain was inoculated into a seed medium, and cultured under shaking at 30°C for 2 days. Then, the above seed culture was inoculated into each flask of a production medium (total 600 mL, each 30 each mL) in an amount of 2 mL to each, and cultured under shaking at 25°C for 5 days.

### (Purification)

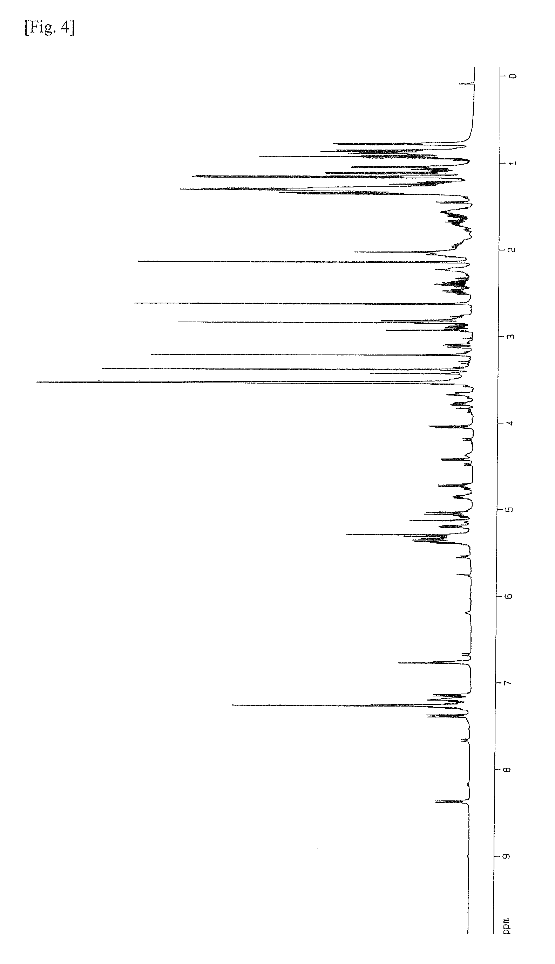

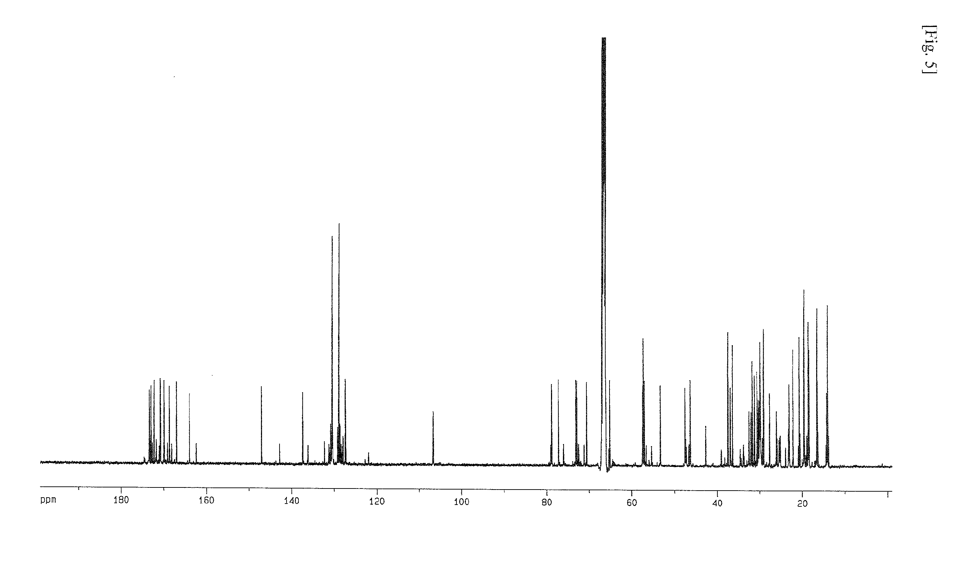

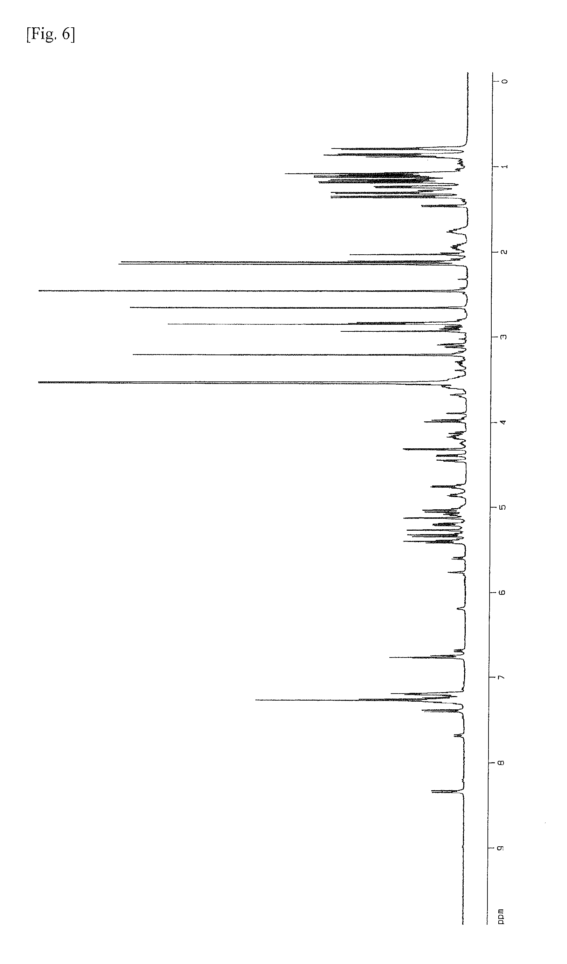

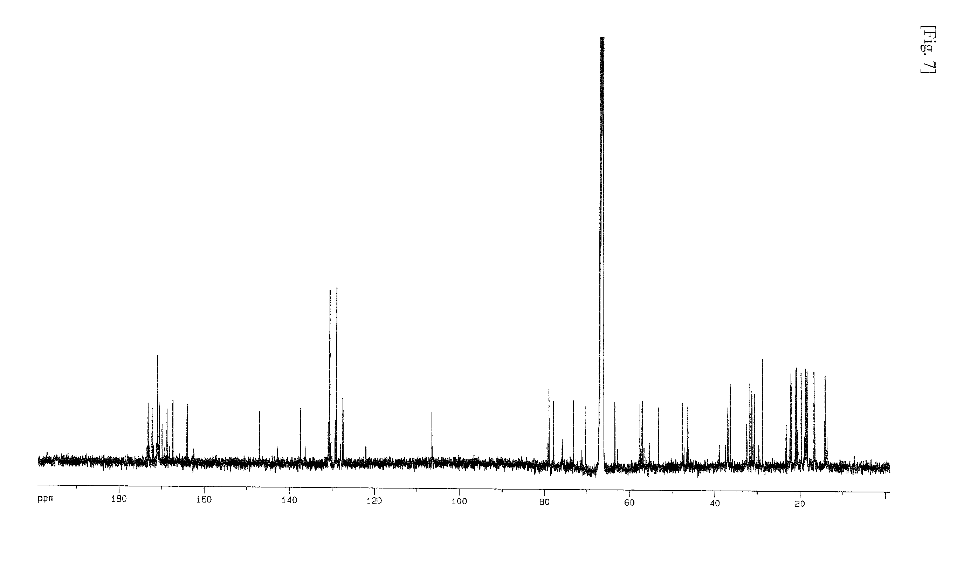

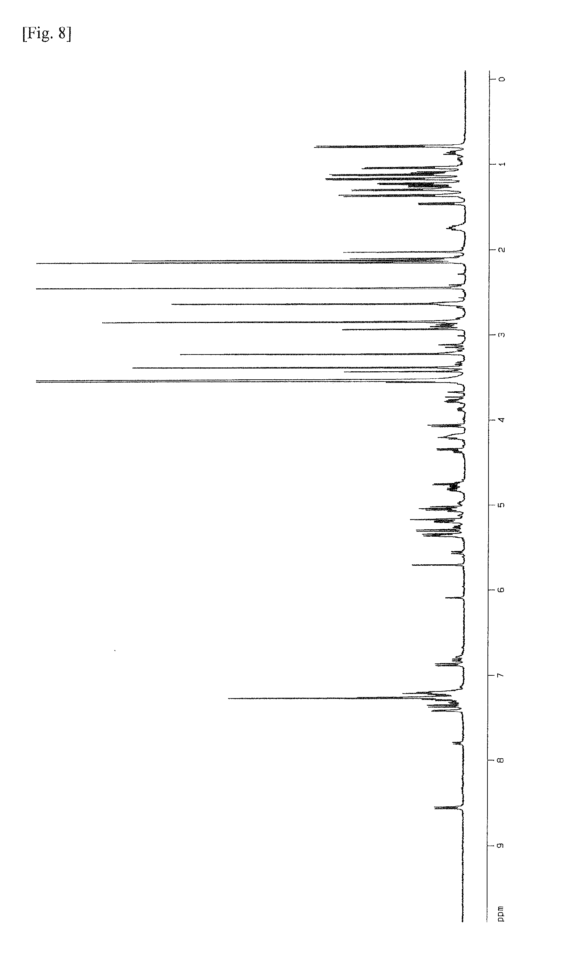

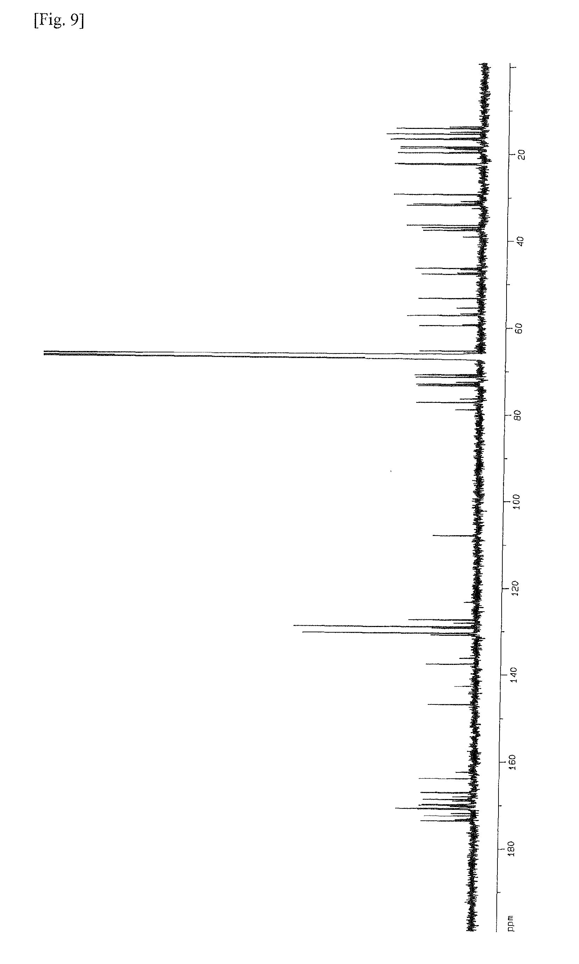

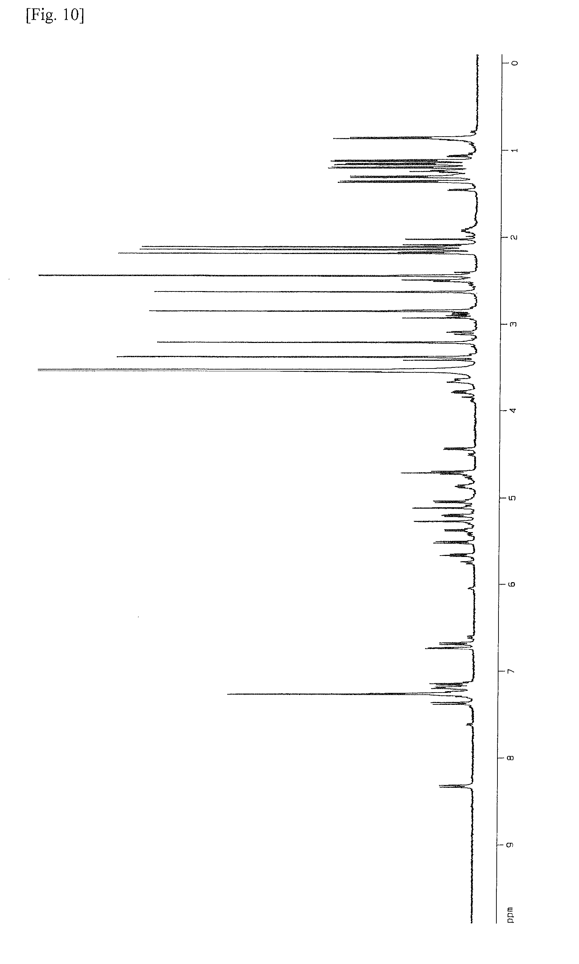

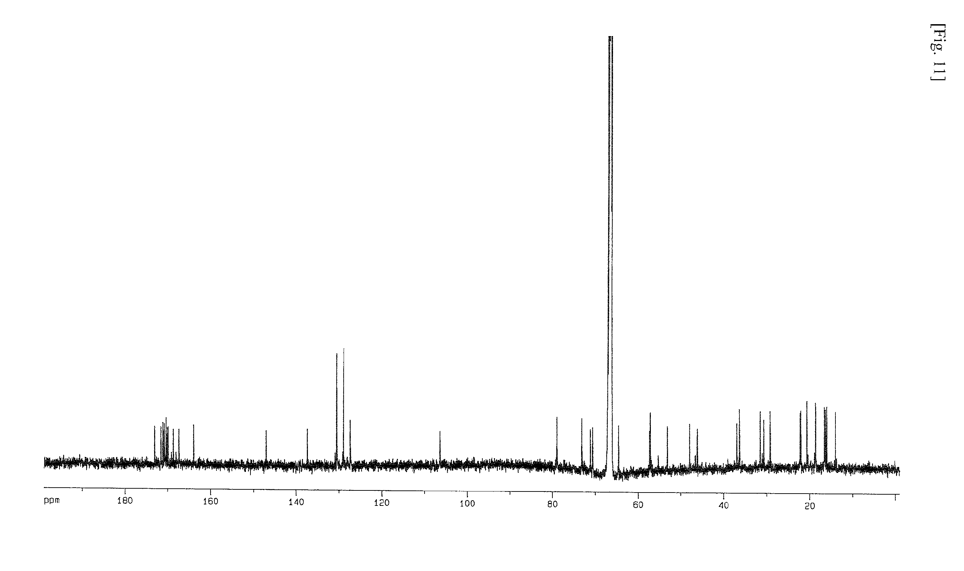

600 mL of acetone was added to 600 mL of the culture, the cells were disrupted, and then the cells were removed by filtration. Then, after concentrating the filtrate until it became an aqueous solution, extraction was carried out twice with 600 mL of EtOAc. 7.1 g of this EtOAc extract was subjected to column chromatography using a Daiso Gel SP-120-15/30-ODS-B (20x250 mm) and eluted with MeOH/water (3:7, 4:6, 5:5, 6:4, 7:3, 8:2, 9:1, 10:0). A fraction 1 eluted with MeOH/water (8:2) was concentrated to dryness in a rotary evaporator, dissolved in 1 mL of MeOH, and purified by CAPCELL PAK MGII 20×250 mm (Shiseido) and HPLC (flow rate 10 mL/min) using MeOH/water (75:25) to obtain 6 mg of Example Compound 1 (Ex1, retention time 41.4 min). A fraction 2 eluted with MeOH/water (7:3) was concentrated to dryness in a rotary evaporator, dissolved in 1 mL of MeOH, and purified by CAPCELL PAK MGII 20x250 mm (Shiseido) and HPLC (flow rate 10 mL/min) using MeOH/water (75:25) to obtain 1 mg of Example Compound 2 (Ex2, retention time 16.0 min), 1 mg of Example Compound 3 (Ex3, retention time 18.4 min), and 1 mg of Example Compound 4 (Ex4 retention time 19.2 min). The physical properties data of Ex1 to Ex4 are shown in the Table below. In addition, the ¹H-NMR spectrum (measurement solvent: 1,4-Dioxane-d₈) and the ¹³C-NMR spectrum (measurement solvent: 1,4-Dioxane-d₈) of each compound are shown, respectively, in Figs. 4 to 11.

**[Table 3]**

| Ex1 | |
|---|---|
| Appearance | White powder |
| Molecular weight | 987 |
| Molecular formula | C₄₈H₇₃N₇O₁₅ |
| HRFAB-MS (m/z) | |
| Found: | 988.5243 (M+H)⁺ |
| Calcd: | 988.5243 |
| [α]²⁵_{D} | - 94.0° (c 0.10, MeOH) |
| UV (MeOH) | end absorption |
| IR υ max (KBr) cm⁻¹ | 3430, 3330, 2970, 2940, 1750, 1680, 1640, 1530 |
| | 1450, 1380, 1280, 1210, 1160, 1100, 1070 |

| Ex2 | |
|---|---|
| Appearance | White powder |
| Molecular weight | 945 |
| Molecular formula | C₄₅H₆₇N₇O₁₅ |
| HRFAB-MS (m/z) | |
| Found: | 946.4770 (M+H)⁺ |
| Calcd: | 946.4774 |
| [α]²⁵_{D} | - 104.4° (c 0.10, MeOH) |
| UV (MeOH) | end absorption |
| IR υ max (KBr) cm⁻¹ | 3430, 3350, 2970, 2940, 1750, 1640, 1530 |
| | 1450, 1410, 1370, 1280, 1230, 1160, 1070 |

| Ex3 | |
|---|---|
| Appearance | White powder |
| Molecular weight | 977 |
| Molecular formula | C₄₅H₆₇N₇O₁₅S |
| HRFAB-MS (m/z) | |
| Found: | 978.4493 (M+H)⁺ |
| Calcd: | 978.4495 |
| [α]²⁵_{D} | - 114.0° (c 0.10, MeOH) |
| UV (MeOH) | end absorption |
| IR υ max (KBr) cm⁻¹ | 3420, 3330, 2980, 2940, 1750, 1680, 1640, 1530 |
| | 1450, 1380, 1280, 1220, 1170, 1070 |

| Ex4 | |
|---|---|
| Appearance | White powder |
| Molecular weight | 977 |
| Molecular formula | C₄₅H₆₇N₇O₁₅S |
| HRFAB-MS (m/z) | |
| Found: | 978.4500 (M+H)⁺ |
| Calcd: | 978.4495 |
| [α]²⁵_{D} | -75.6° (c 0.10, MeOH) |
| UV (MeOH) | end absorption |
| IR υ max (KBr) cm⁻¹ | 3420, 3330, 2980, 2940, 1750, 1640, 1530 |
| | 1450, 1380, 1280, 1220, 1180, 1100, 1070 |

From the above physical property data and NMR data, the planar structural formulae of the Example compounds 1, 2, 3, and 4 were each determined to be the following formulae. It is considered, but not confirmed, that the steric structures include compounds having structures like the formula (IIa). In addition, Ex3 and Ex4 have the relation of optical isomers with each other, but the position of an asymmetric carbon forming the isomers is uncertain. 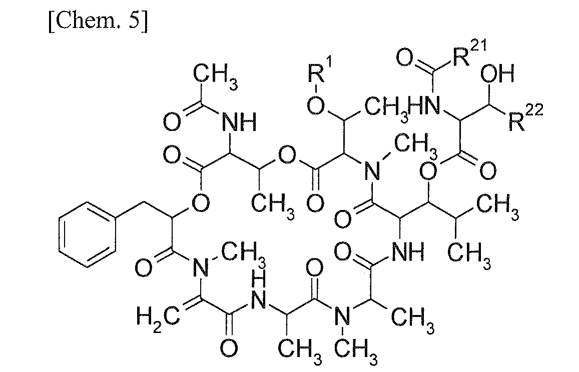 EX1: R¹=-CH₃, R²¹=-CH₂CH₂CH₃, R²²=-CH(CH₃)₂
Ex2: R¹=-H, R²¹=-CH₃, R²²=-CH(CH₃)₂
Ex3: R¹=-CH₃, R²¹=-CH3, R²²=-CH₂SCH₃
Ex4_{:} R¹=-CH₃, R²¹=-CH₃, R²²=-CH₂SCH₃

The pharmacological activity of the compound of the formula (II) was confirmed by the tests shown below.

### Test Example 1: Inhibitory action on G_{q/11}

### (Method)

The test was carried out according to the method of Non-Patent Citation 3, using a leukotriene receptor CysLTR2 (cysteinyl leukotriene receptor 2), which is known to activate G_{q/11}, CHO cells stably expressing CysLTR2 were seeded on a 96-well black/clear bottom plate (manufactured by Becton, Dickinson and Company) at 20,000 cells per well, and after 24 hours of seeding, the medium was discarded. Then, Hanks BSS (GIBCO Inc.) including 2 µM Fluo-4, AM (manufactured by Molecular Probe Co.), 0.004% pluronic acid, 1% FBS, 20 mM HEPES, and 2.5 mM probenecid was added thereto at 100 µL per well, followed by incubation at 37°C for 1 hour. After incubation, the resultant was washed four times with Hanks BSS including 20 mM HEPES, and Hanks BSS including 20 mm HEPES per well was added thereto 100 µL per well. In addition, 50 µL of a test compound that had been diluted with Hanks BSS including 20 mM HEPES to a final concentration from 1×10⁻¹⁰ M to 1×10⁻⁵ M was added thereto, followed by being left to stand at room temperature for 30 minutes. The change of cellular Ca²⁺ concentration after addition of leukotriene D₄ (LTD₄, manufactured by Cayman Co.), which is a CysLTR2 ligand, was measured over time using an FLIPR (Molecular Device Corp.). 50 µL of leukotriene D₄ that had been diluted with Hanks BSS including 20 mM HEPES to a final concentration of 10 nM at 10 seconds after the initiation of measurement was added thereto, fluorescence intensities were measured every second for 50 seconds and every sixth second for 2 minutes, and a maximum fluorescence intensity during a measurement time of 3 minutes was observed. By plotting the concentrations and the fluorescence intensities of the test compounds, the IC₅₀ values were calculated, which were taken as G_{q/11} inhibitory activities. As a result, for example, the G_{q/11} inhibitory activities (IC₅₀ values) of Ref 1, Ref 2, Ref 3, Ref 5, and Ref 7 were 66, 34, 56, 25, and 32 (nM), respectively, and the G_{q/11} inhibitory activities (IC₅₀ values) of Ex1, Ex2, Ex3, and Ex4 were 31, 317, 97, and 365 (nM), respectively.

From the results of Test Example 1, it was confirmed that the compound of the present invention has a good G_{q/11} inhibitory action.

### Test Example 2: Inhibitory action of intratracheal administration on the methacholine-induced airway contraction

### (Method)

SD male rats (Charles River Laboratories Japan, Inc.) were used in experiments. Urethane (1.2 g/kg) was administered intraperitoneally to the rats for anesthesia. Using a metal canula for intratracheal administration, test compounds (0.3, 1, and 3 µg/kg) or physiological saline was administered intratracheally (3 or 4 animals) at a volume of 0.5 mL/kg. Cannulation for intravenous administration was carried out in the external jugular vein of the rats. A tracheostomy cannula was inserted through an incision in the trachea and mechanical ventilation (10 mL/kg, 90 times/min) was carried out using a Breathing Analysis Computer System (flexiVent, SCIREQ Inc.). The mechanical ventilator circuit was connected with a pressure transducer (TP-400T, Nihon Kohden Corporation) and the change in airway pressure was measured. The airway pressure signal was amplified with a strain pressure amplifier (AP-601G, Nihon Kohden Corporation) and recorded on chart paper by a thermal pen recorder (WT-685G, Nihon Kohden Corporation, or LINEARCORDER F WR3701, Graphtec). The body temperature of the rats was maintained at 37°C using a body temperature-maintaining device (BWT-100, Bio Research Center). At 1 hour after intratracheal administration of the test compound, a solution of acetyl-β-methacholine chloride in physiological saline (30 µg/kg, acetylcholine β-methacholine chloride=manufactured by SIGMA Co.) was administered intravenously to induce the airway contraction response. The airway contraction inhibitory rate of the test compound was calculated by comparing the airway pressure increase rate (%) with one obtained by intratracheal administration of physiological saline, and ED₅₀ values were determined.

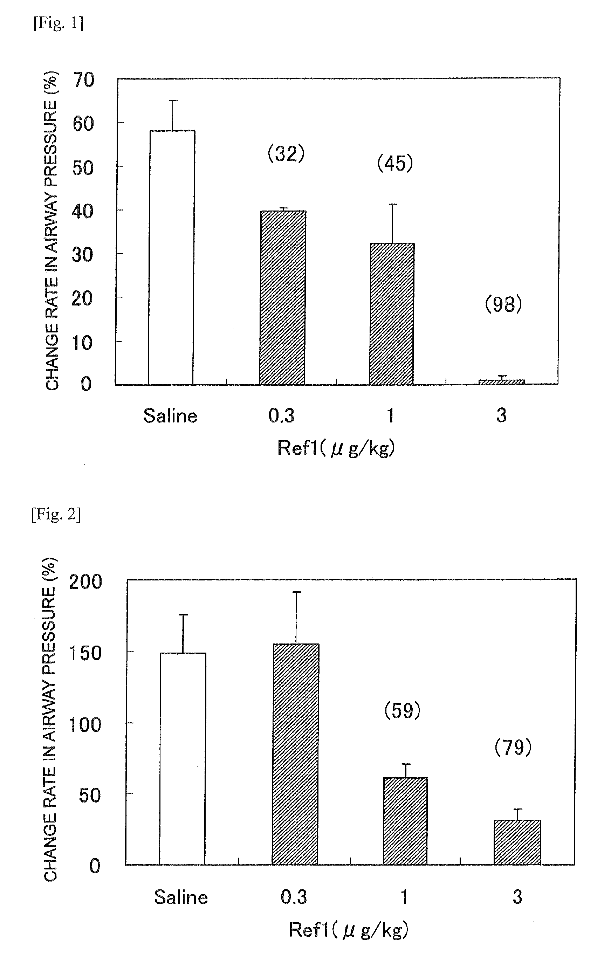

Fig. 1 shows the percentage change rate in airway pressures of Ref 1. Ref 1 showed inhibitory rates of 32, 45, and 98%, respectively, by intratracheal administration to rats at 0.3, 1, and 3 µg/kg at 1 hour before induction of airway contraction. The ED₅₀ value calculated from three points was 0.73 µg/kg. In addition, in the same manner as in Ref2 to 7 and Ex 1 to 4, the airway contraction response was inhibited at a dose ranging from 1 µg/kg to 10 µg/kg.

From the results of Test Example 2, it was confirmed that the methacholine-induced airway contraction response was inhibited by intratracheal administration of the compound of the present invention to rats.

### Test Example 3: Action on the LTD₄-induced airway contraction response by intratracheal administration

### (Method)

Hartley male guinea pigs (Japan SLC, Inc.) were used in experiments. Urethane (1.2 g/kg) was administered intraperitoneally to the guinea pigs for anesthesia. Using needles (30G), the test compounds (0.3, 1, and 3 µg/kg) at a volume of 0.5 mL/kg or physiological saline was administered by intratracheal injection (3 animals in each group). Cannulation for intravenous administration was carried out in the external jugular vein of the guinea pigs. A tracheostomy cannula was inserted through an incision in the trachea and mechanical ventilation (10 mL/kg, 60 times/min) was carried out using a mechanical ventilator (MODEL683, HARVARD). The mechanical ventilator circuit was connected with a pressure transducer (TP-400T, Nihon Kohden Corporation) and the change in airway pressure was measured. The airway pressure signal was amplified with a strain pressure amplifier (AP-601G, Nihon Kohden Corporation) and recorded on chart paper by a thermal pen recorder (LINEARCORDER F WR3701, Graphtec). The body temperature of the guinea pigs was maintained at 37°C using a body temperature-maintaining device (BWT-100, Bio Research Center). At 30 minutes after injection by intratracheal administration of the test compound, a solution of LTD₄ in physiological saline (0.3 µg/kg) was administered intravenously to induce the airway contraction response.

### (Results)

The percentage change rate in airway pressures of Ref 1 are shown in Fig. 2. For the group administered with physiological saline, a remarkable airway contraction response was exhibited, while for the group administered with Ref 1, it was construed that the airway contraction response was inhibited (ED₅₀ value of 1.1 µg/kg).

From the results of Test Example 3, it was confirmed that the LTD₄-induced airway contraction response was inhibited by intratracheal administration of the compounds of the present invention.

### Test Example 4: Action on the carotid pressure by intratracheal administration

### (Method)

SD male rats (Charles River Laboratories Japan, Inc.) were used in experiments. Urethane (1.2 g/kg) was administered intraperitoneally to the rats for anesthesia. Cannulation for intravenous administration was carried out in the carotid artery and a pressure transducer (TP-400T, Nihon Kohden Corporation) was connected. The cannula for measuring blood pressure and a pressure transducer were filled with 5% heparin physiological saline. The pressure signal was amplified with a strain pressure amplifier (AP-601 G, Nihon Kohden Corporation) and recorded on chart paper by a thermal pen recorder (WT-685G, Nihon Kohden Corporation). The body temperature of the rats was maintained at 37°C using a body temperature-maintaining device (BWT-100, Bio Research Center). The trachea was exposed, and using needles (27G), the test compounds (30 or 100 µg/kg) at a volume of 0.5 mL/kg or physiological saline was administered by direct intratracheal injection. For the group administered with 30 µg/kg, 5 animals were used, for the group administered with 100 µg/kg, 1 animal was used, and for the group administered with physiological saline, 3 animals were used. For 30 minutes after intratracheal administration, a mean blood pressure was measured.

### (Results)

Reductions in the blood pressure 10 minutes after intratracheal administration to the group, for the group administered intratracheally with physiological saline or Ref 1 at a dose of 30 µg/kg, were 5±2.4 mmHg and 7±S.2 mmHg, respectively, and the maximum reductions during 30 minutes for observation of the blood pressure were 20±7.1 mmHg and 22±3.3 mmHg, respectively, and there was no significant difference between them. On the other hand, for the group administered intratracheally with Ref 1 at a dose of 100 µg/kg, it was considered that there was remarkable reduction in blood pressure.

**[Table 41**

| | | | Reduction in blood pressure (mmHg) | |
|---|---|---|---|---|
| Drug | Dose | Number of animals | After 10 min | Max in 30 min |
| Saline | - | 3 | 5±2.4 | 20±7.1 |
| Ref 1 | 30 µg/kg | 5 | 7±5.2 | 22±3.3 |
| | 100 µg/kg | 1 | 65 | 95 |

From the results of Test Example 4, it was suggested that the amount having no action on the carotid artery pressure when Ref 1 was administered intratracheally to the rats was at least 30 µg/kg. From the results, it was suggested that a maximum amount having no action on the carotid artery pressure (at least 30 µg/kg) and the ED₅₀ value (0.73 µg/kg) of the inhibitory action on airway contraction had a gap of at least 40 times between them.

### Test Example 5: Action of nasal drop administration on the tobacco smoke inhalation-induced airway inflammation

### (Method)

BALB/c male mice (Charles River Laboratories Japan, Inc.) were used in experiments. Sodium pentobarbital (40 mg/kg) was administered intraperitoneally to the mice for anesthesia. During a period of time ranging from Day 0 to Day 10 (except for Days 5 and 6), the test compound (1, 10, and 100 µg/kg) were administered nasally to the mice once a day. The Vehicle group was similarly injected with physiological saline by nasal drop administration. There were 10 animals in each group. The mice were put into chambers at 1 hour after nasal drop administration, and using a tobacco smoke generator (SG-200, Sibata Scientific Technology Ltd.), the tobacco smoke was inhaled to a concentration in air up to 4% for 1 hour. After intraperitoneal administration of sodium pentobarbital (80 mg/kg) to the mice at Day 11, the mice were subject to blood removal to be euthanized. After respiratory arrest, the trachea was incised, bronchoalveolar lavage was performed with PBS(-) 0.5 mLx3 times, and the washing liquid was collected. The supernatant was removed by centrifugation, the cells were then resuspended in PBS(-), and for white blood cells (WBC), neutrophils (NEUT), lymphocytes (LYMPH), macrophages (MONO), and eosinophils (EO), the cell count was measured with a Multiple Parameter Automatic Hematology Analyzer (XT-2000i, Sysmex Corporation).

### (Results)

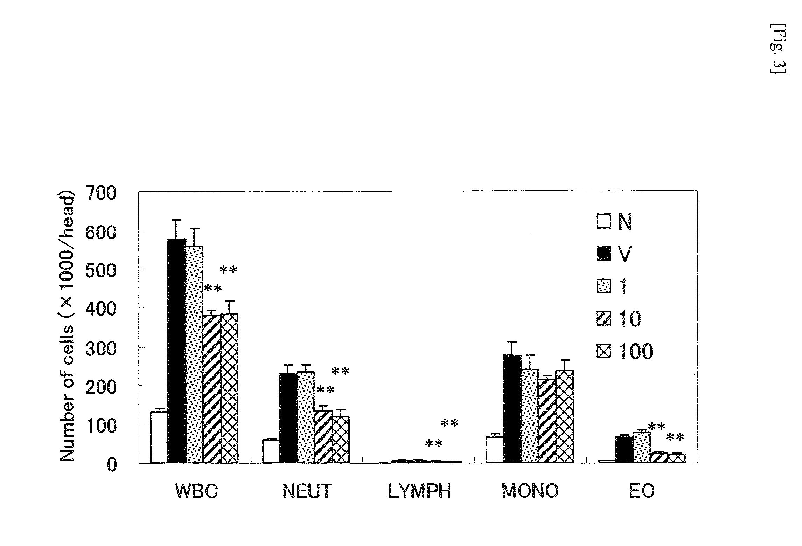

The results of Ref 1 are shown in Fig. 3. Ref 1 significantly inhibited infiltration of neutrophils, lymphocytes, and eosinophils into the airway when being injected at doses of 1, 10, and 100 µg/kg once a day by nasal drop administration.

From the results of Test Example 5, it was confirmed that the compound of the present invention inhibits infiltration of neutrophils, lymphocytes, and eosinophils into the airway induced by inhalation of tobacco smoke.

From the results of Test Example 1, it was confirmed that the compound of the present invention has a G_{q/11} inhibitory action, and from the results of Test Example 2 and Test Example 3, it was suggested that the compound of the present invention has an inhibitory action on the methacholine- or LTD₄-induced airway contraction by intratracheal administration. From Test Example 4, it was confirmed that the maximum amount having no action on the carotid artery pressure has a large difference from the ED₅₀ value showing an inhibitory action on airway contraction. Furthermore, from Test Example 5, it was confirmed that the compound has an action of inhibiting infiltration of neutrophils, lymphocytes, and eosinophils into the airway induced by inhalation of tobacco smoke, and thus has an inhibitory action on airway inflammation. From the results above, it is suggested that the compound of the present invention has both an inhibitory action on the airway contraction and an inhibitory action on the airway inflammation, when being received by intratracheal, nasal drop, or inhalation administration, and since there is a possibility of these actions having a gap from the blood pressure-reducing action, it is expected that that the compound of the present invention is useful for prevention or treatment of COPD or asthma.

### Industrial Applicability

The cyclic depsipeptide compound or a salt thereof of the present invention has a G_{q/11} inhibitory action, has an inhibitory action on airway contraction and an inhibitory action on airway inflammation, and thus, is useful for prevention and/or treatment of COPD, asthma, or the like.

## Claims

1. A cyclic depsipeptide compound of the formula (I) or a salt thereof: 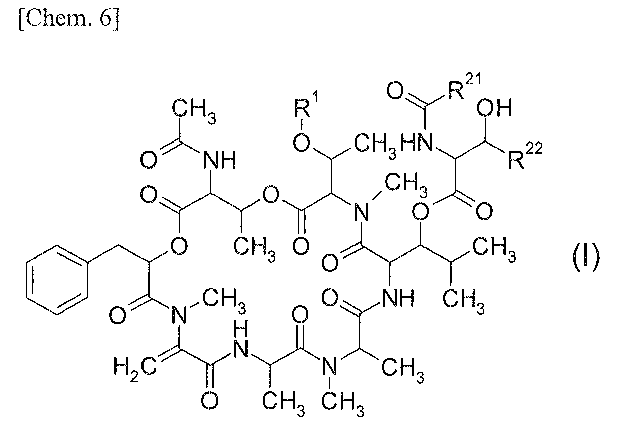 (wherein
R¹ is -CH₃, R²¹ is -CH₂CH₂CH₃, and R²² is -CH(CH₃)₂;
R¹ is -H, R²¹ is -CH₃, and R²² is -CH(CH₃)₂; or
R¹ is -CH₃, R²¹ is -CH₃, and R²² is -CH₂SCH₃).

2. A pharmaceutical composition for preventing and/or treating chronic obstructive pulmonary disease (COPD) or asthma, which comprises a cyclic depsipeptide compound of the formula (II) or a salt thereof as an active ingredient and is intended to be administered by intratracheal administration, nasal drop administration, or inhalation administration: 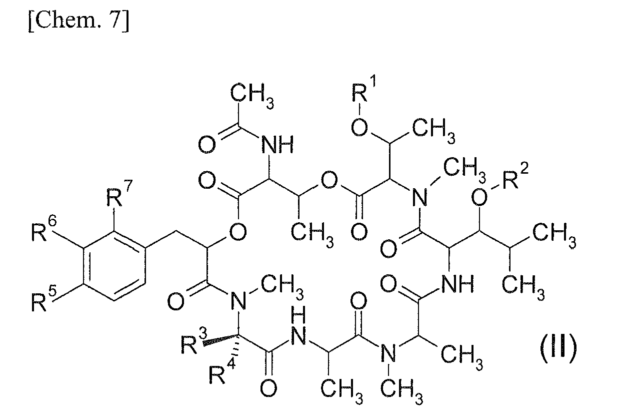 [wherein
R¹ is -H or -CH₃, and
R² is -H or a group represented by the formula (III): 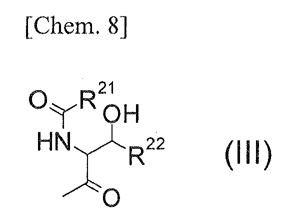 R²¹ is -CH₃, -CH₂CH₃, -CH₂CH₂CH₃, or -CH₂SCH₃,
R²² is -CH(CH₃)₂ or -CH₂SCH₃,
R³ and R⁴ are the same as or different from each other, and represent -H or -CH₃, or R³ and R⁴ are combined to form =CH₂, and
R⁵, R⁶, and R⁷ are the same as or different from each other, and represent -H or -F].

3. The pharmaceutical composition as described in claim 2, wherein the cyclic depsipeptide compound or a salt thereof is a cyclic depsipeptide compound represented by either the formula (IIa) or the formula (I), or a salt thereof:
**[Table 5]**
| | | | | | | | | |
|---|---|---|---|---|---|---|---|---|
| 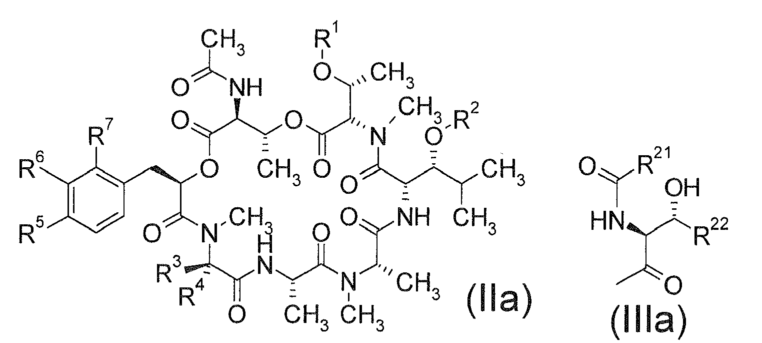 | | | | | | | | |
| (wherein R¹, R², R²¹, R²², R³, R⁴, R⁵, R⁶, and R⁷ are any one of combinations in the Table below) | | | | | | | | |
| R¹ | R² | R²¹ | R²² | R³ | R⁴ | R⁵ | R⁶ | R⁷ |
|---|---|---|---|---|---|---|---|---|
| -CH₃ | (IIIa) | -CH₃ | -CH(CH₃)₂ | =CH₂ | | -H | -H | -H |
| -CH₃ | (IIIa) | -CH₂CH₃ | -CH(CH₃)₂ | =CH₂ | | -H | -H | -H |
| -CH₃ | (IIIa) | -CH₂SCH₃ | -CH(CH₃)₂ | =CH₂ | | -H | -H | -H |
| -CH₃ | -H | - | - | =CH₂ | | -H | -H | -H |
| -CH₃ | (IIIa) | -CH₃ | -CH(CH₃)₂ | =CH₂ | | -H | -H | -F |
| -CH₃ | (IIIa) | -CH₃ | -CH(CH₃)₂ | =CH₂ | | -H | -F | -H |
| -CH₃ | (IIIa) | -CH₃ | -CH(CH₃)₂ | -CH₃ | -H | -H | -H | -H |
| 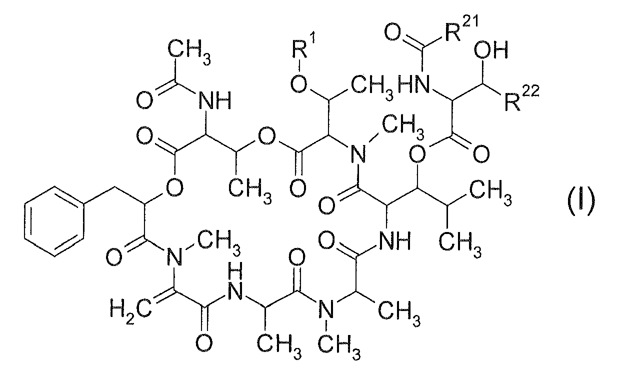 | | | | | | | | |
| (wherein R¹, R²¹, and R²² are any one of combinations in the Table below) | | | | | | | | |
| R¹ | | R²¹ | R²² | | | | | |
|---|---|---|---|---|---|---|---|---|
| -CH₃ | | -CH₂CH₂CH₃ | -CH(CH₃)₂ | | | | | |
| -H | | -CH₃ | -CH(CH₃)₂ | | | | | |
| -CH₃ | | -CH₃ | -CH₂SCH₃ | | | | | |

4. A cyclic depsipeptide compound of the formula (II) or a salt thereof for prevention or treatment of COPD or asthma by intratracheal administration, nasal drop administration, or inhalation administration: 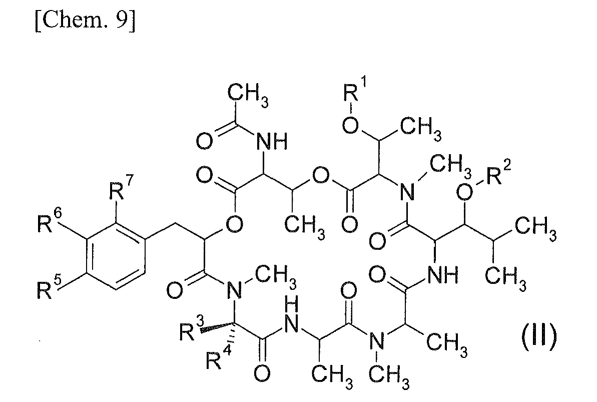 [wherein
R¹ is -H or -CH₃,
R² is -H or a group represented by the formula (III): 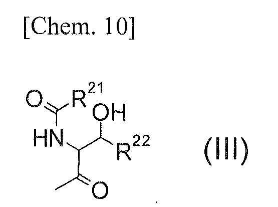 R²¹ is -CH₃, -CH₂CH₃, -CH₂CH₂CH₃, or -CH₂SCH₃,
R²² is -CH(CH₃)₂ or -CH₂SCH₃,
R³ and R⁴ are the same as or different from each other, and represent -H or -CH₃, or R³ and R⁴ are combined to form =CH₂, and
R⁵, R⁶ and R⁷ are the same as or different from each other, and represent -H or -F].

5. A method for preventing or treating COPD or asthma, comprising administering to a patient in need thereof an effective amount of the cyclic depsipeptide compound of the formula (II) or a salt thereof by any one process of intratracheal administration, nasal drop administration, and inhalation administration: 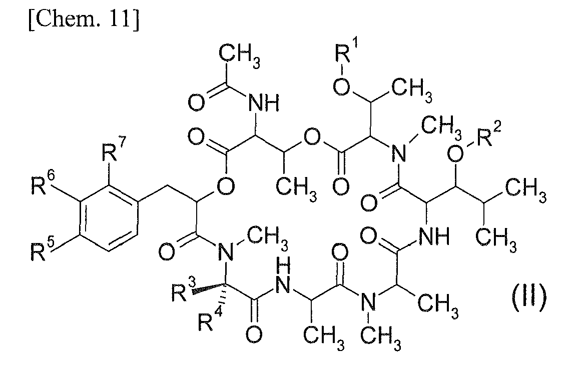 [wherein
R¹ is -H or -CH₃,
R² is -H or a group represented by the formula (III): 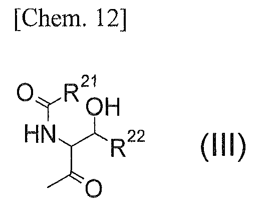 R²¹ is -CH₃, -CH₂CH₃, -CH₂CH₂CH₃, or -CH₂SCH₃,
R²² is -CH(CH₃)₂ or -CH₂SCH₃,
R³ and R⁴ are the same as or different from each other, and represent -H or -CH₃, or R³ and R⁴ are combined to form, =CH₂, and
R⁵, R⁶ and R⁷ are the same as or different from each other, and represent -H or -F].
